Europäisches Patentamt

European Patent Office

Office européen des brevets

(11) Veröffentlichungsnummer: **0 185 374**
**B1**

(12) **EUROPÄISCHE PATENTSCHRIFT**

(45) Veröffentlichungstag der Patentschrift: 02.05.90

(51) Int. Cl.⁵: **A 61 K 31/19, A 61 K 47/00**

(21) Anmeldenummer: 85116219.8

(22) Anmeldetag: 19.12.85

(54) Flüssige Diclofenac-Zubereitungen.

(30) Priorität: 21.12.84 DE 3446873

(43) Veröffentlichungstag der Anmeldung:
25.06.86 Patentblatt 86/26

(45) Bekanntmachung des Hinweises auf die
Patenterteilung:
02.05.90 Patentblatt 90/18

(84) Benannte Vertragsstaaten:
AT BE CH DE FR GB IT LI LU NL SE

(56) Entgegenhaltungen:
EP-A-0 136 470
DE-A-2 055 795
DE-A-2 302 659

CHEMICAL ABSTRACTS, Band 101, Nr. 2, Juli
1984, Seite 305, Zusammenfassung Nr. 12213p,
Columbus, Ohio, US; & JP-A-59 33 211 (SATO
PHARMACEUTICAL CO., LTD) 23-02-1984

(73) Patentinhaber: Merckle GmbH
Dr.-Georg-Spohn-Strasse 7
D-7902 Blaubeuren (DE)

(72) Erfinder: VON Stetten, Otto, Dr.
Blaubeurerstrasse 91
D-7933 Schelklingen (DE)
Erfinder: Seth, Pyare Lal, Dr.
Krebsenbachweg 8
CH-4147 Aesch/BL (CH)
Erfinder: Schmid, Franz
Mozartstrasse 48
D-8068 Pfaffenhofen (DE)
Erfinder: Räuchle, Kurt, Dr.
Forstweg 16
D-7902 Blaubeuren (DE)

(74) Vertreter: Lederer, Franz, Dr. et al
Van der Werth, Lederer & Riederer
Patentanwälte Lucile-Grahn-Strasse 22
D-8000 München 80 (DE)

**Beschreibung**

Die Erfindung betrifft stabile flüssige Diclofenac-Zubereitungen, die besonders für parenterale Wirkstoffapplikation geeignet sind. Diclofenac ist der international eingeführte Trivialname des Wirkstoffes [2-(2,6-Dichlor-anilino)-phenyl]essigsäure; es ist ein nicht-steroidales Antirheumatikum, das zur symptomatischen Therapie bei Rheumaerkrankungen eingesetzt wird. Wegen des relativ großen "firstpass-effects" der Substanz und zur rascheren Anflutung ist es wünschenswert, Injektionslösungen einzusetzen, wobei eine Dosis von 75 mg pro Injektion verwendet werden soll. Für intramuskuläre Injektion soll das Volumen jedoch möglichst niedrig gehalten werden.

Wegen der relativen Schwerlöslichkeit von Diclofenac in Wasser ist eine wäßrige Injektionslösung mit vernünftigem Volumen nicht zu erhalten. Außerdem ist Diclofenac in Lösung relativ instabil. Aufgabe der vorliegenden Erfindung war es daher, injizierbare Diclofenac-Zubereitungen zur Verfügung zu stellen, die bei kleinem Injektionsvolumen die möglichst schmerzfreie Injektion therapeutisch ausreichender Arzneistoffmengen zulassen und außerdem lagerstabil sind.

Die US—P 3 652 762 offenbart wässrige, Propylenglykolhaltige Sonnenschutzcremes, die etwa 1 Gew.-% Diclofenac enthalten.

Bisher verfügbare Diclofenac-Injektionslösungen enthalten die gewünschte Applikationsmenge von 75 mg Diclofenac-Natrium in 3 ml Lösung, ihr Wirkstoffgehalt beträgt also 2,5%. Ihr pH-Wert beträgt pH 8,5. Als Lösungsvermittler bzw. Konservierungsstoff sind Propylenglykol und Mannit bzw. Sorbit und Benzylalkohol, sowie als Stabilisator Sulfit eingesetzt worden. Zusätze von Lokalanästhetika in therapeutisch wirksamer Menge zur Verhinderung von Injektionsschmerzen sind in den bekannten Präparaten nicht enthalten, in den bislang verwendeten Lösungsmittelsystemen aus Löslichkeitsgründen auch nicht möglich. Ebensowenig ist es möglich, zusätzlich weitere Arzneistoffe, z.B. Steroide als zusätzliche antiinflammatorisch wirksame Substanzen in die Lösung einzubringen, wenn das Injektionsvolumen in vertretbaren Grenzen bleiben soll.

Propylenglykol und Polyethylenglykol sind als Lösungsvermittler bekannt. Es wurde daher zunächst versucht, Diclofenac in einer Mischung aus Wasser und Propylenglykol in Lösung zu bringen, jedoch zeigte ein solches System keine ausreichende Lösungseigenschaften. Bei Verwendung von Polyethylenglykol anstelle von Propylenglykol wurde zwar eine bessere Auflösung erreicht, jedoch war die Lösung zu viskos, um für eine parenterale Applikation verwendet zu werden.

Es wurde nun überraschend gefunden, daß mit einem Lösungsmittelsystem bestehend aus Wasser und einem Gemisch aus Propylenglykol und Polyethylenglykol in den nachstehend angegebenen Mengenverhältnissen vergleichsweise hochkonzentrierte Wirkstofflösungen zumindest bei höheren pH-Werten erhalten werden können.

Darüber hinaus wurde überraschenderweise festgestellt, daß eine noch bessere Lösung des Diclofenacs erreicht werden kann, wenn dem System zusätzlich ein Lokalanästhetikum, nämlich Lidocain, zugesetzt wird. Hierdurch wird eine ausreichende Lösungsfähigkeit schon bei tieferen, dem physiologischen Wert mehr entsprechenden pH-Werten erreicht.

Die meisten Lokalanästhetika sind nämlich wie das Lidocain leicht basische Substanzen, die mit Säuren wie HCl Salze bilden. Die Lokalanästhetika werden zweckmäßigerweise in Form ihrer Säureadditionssalze, insbesondere als Hydrochloride eingesetzt. Lokalanästhetika von der Art des Lidocains sind insbesondere Bupivacain, Etidocain, Pyrrocain und Mepivacain.

Die Wirkung des Lidocains ist insbesondere deshalb überraschend, weil zuvor festgestellt wurde, daß das Lösungsmittelsystem bei höherem pH-Wert besser wirkt als bei tieferem pH-Wert und der Zusatz von Lidocain-HCl den pH-Wert sicher nicht erhöht, sondern erniedrigt.

Es wurde weiter gefunden, daß der Zusatz von Lidocain nicht nur die Löslichkeit, also die physikalische Stabilität, sondern auch die chemische Stabilität der Lösungen verbessert, wenn gleichzeitig ein Reduktionsmittel zugesetzt wird.

Erfindungsgemäß ist es also möglich, die gewünschte Applikationsmenge von 75 mg in nur 2 ml Injektionslösung in stabiler Lösung zu halten.

Gegenstand der Erfindung sind somit flüssige Diclofenac-Zubereitungen, insbesondere für die parenterale Applikation, bestehend aus einer Lösung von Diclofenac oder eines seiner Salze und gegebenenfalls weiteren pharmazeutischen Wirkstoffen und Hilfsstoffen in einem Lösungsmittel, wobei das Lösungsmittel aus 10—70 Gew.-%, vorzugsweise 20—50 Gew.-%, einer Mischung aus a) Propylenglykol und b) Polyethylenglykol und 90—30 Gew.-%, vorzugsweise 80—50 Gew.-% Wasser besteht, und in dem Lösungsmittelgemisch das Gewichtsverhältnis von Propylenglykol : Polyethylenglykol zwischen 9,5 : 0,5 und 0,5 : 9,5, vorzugsweise zwischen 3 : 1 und 1 : 3, besonders bevorzugt zwischen 2 : 1 und 1 : 2 liegt.

Im allgemeinen enthalten die Zubereitungen 1,5 bis 6 Gew.-% Diclofenac, vorzugsweise 3—4 Gew.-% Diclofenac-Natrium.

Als Polyethylenglykol wird vorzugsweise Polyethylenglykol 400 eingesetzt.

Die Konzentration eines zugesetzten Lokalanästhetikums, insbesondere Lidocains, kann zwischen 0,1 Gew.-% und 5 Gew.-% schwanken, und liegt bevorzugt im Bereich von 0,5 Gew.-% bis 2 Gew.-% bezogen auf Lidocain-HCl. Der Zusatz eines Lokalanästhetikums ist auch aus medizinischen Gründen zur Verhinderung des Injektionsschmerzes nicht unerwünscht.

2

Bei den erfindungsgemäßen Zubereitungen ist es möglich, den pH-Wert im Bereich zwischen pH = 6,5 und pH = 8 einzustellen.

Zur chemischen Stabilisierung wird den Lösungen außerdem ein Reduktionsmittel als Stabilisator zugesetzt. Dabei sind endogene Substanzen wie Cystein oder Thiosulfat vorzuziehen. Bei üblichen Lösungsmitteln entsteht bei Verwendung von Cystein häufig ein nicht näher definierbarer Niederschlag. Bei den erfindungsgemäßen Zubereitungen tritt ein solcher Niederschlag selbst nach mehrmonatiger Lagerung nicht auf. Bei Verwendung von N-Acetylcystein, einem in höheren Konzentrationen verwendeten pharmazeutisch durchaus annehmbaren Wirkstoff, ist die Möglichkeit von Ausfällungen weiter reduziert.

Bei den erfindungsgemäßen Zubereitungen können als Stabilisatoren Sulfit, Cystein bzw. Cysteinhydrochlorid, Acetylcystein bzw. -Hydrochlorid, Thiosulfat usw., vorzugsweise Sulfit, Cystein, Acetylcystein, ganz besonders bevorzugt Sulfit und Acetylcystein, in Konzentrationen zwischen 0,05 Gew.-% und 5 Gew.-%, vorzugsweise zwischen 0,05 Gew.-% und 3 Gew.-%, zugesetzt werden.

In der Therapie der rheumatischen Erkrankungen ist es häufig angezeigt zur Wirkungsverstärkung neben Diclofenac weitere antiinflammatorische Stoffe, vorzugsweise Steroide, z.B. Glucocorticoide wie Dexamethason oder Prednisolon, zu verabreichen. In üblicherweise verwandten Lösungsmitteln sind Steroide oder geeignete Derivate neben Diclofenac relativ schlecht löslich. Bei den erfindungsgemäßen Zubereitungen ist es jedoch möglich in für i. m. Injektion bevorzugten Volumina, z.B. 2 ml, neben 75 mg Diclofenac-Natrium auch größere Mengen Steroid, z.B. 25 mg Prednisolon in Form von 33 mg Prednisolon-21-phosphat-Dinatriumsalz, lagerstabil zu lösen.

Alle Prozentangaben beziehen sich auf die komplette Injektionslösung.

## Vergleichsversuch

Es wurde versucht, 3,75 Gew.-% Diclofenac-Natrium, 0,1 Gew.-% Natriumdisulfit und 0,01 Gew.-% Ethylendiamintetraessigsäure (EDTA) ohne Zusazt von Lidocain sowie unter Zusatz von 1 Gew.-% Lidocain in Lösungsmittelsystemen aus a) 42 Gew.-% Polyethylenglykol (PEG), Rest Wasser; b) 42 Gew.-% Propylenglykol (PG), Rest Wasser; c) 24 Gew.-% Polyethylenglykol (PEG), 18 Gew.-% Propylenglykol (PG), Rest Wasser, bei pH 7 bzw. pH 8 in stabile Lösung zu bringen. Die Prozentangaben beziehen sich auf die Gesamtmischung. Als Polyethylenglykol (PEG) wurde Polyethylenglykol 400 eingesetzt. Die Einstellung des pH-Wertes erfolgte mit Natronlauge. Die Ampullen wurden einer Wechselbelastung 6 Stunden Raumtemperatur — 6 Stunden Kühlschrank während 14 Tagen ausgesetzt. Die Ergebnisse sind in der nachstehenden Tabelle zusammengefaßt. Dort, wo eine stabile Lösung erhalten wurde, ist dies mit + angezeigt, wo keine stabile Lösung erhalten wurde, ist dies mit — angegeben.

|  | 0% Lidocain | | | 1% Lidocain | | |
|---|---|---|---|---|---|---|
|  | 42% PEG | 42% PG | 24% PEG 18% PG | 42% PEG | 42% PG | 24% PEG 18% PG |
| pH 7 | (+) − * | − | − | + | − | + |
| pH 8 | + | − | + | + | − | + |

Die Ansätze mit 42% PEG waren zu viskos, um für parenterale Applikation verwendet werden zu können.
* Bei der Nachkontrolle nach 14 Tagen wurde eine Kristallbildung beobachtet.

## Beispiel 1

7,5 g Diclofenac-Na werden in einer Lösung aus 48 g Propylenglykol und 36 g Polyethylenglykol 400 gelöst. Es werden 20 mg EDTA, 200 mg Na-disulfit und 2 g Lidocain zugesetzt. Nach Auffüllen mit Wasser auf 195 ml wird mit Natronlauge auf pH 8 eingestellt und auf 200 ml aufgefüllt. Die Lösung wird in Ampullen zu 2 ml abgefüllt und bei 120°C 20 Minuten sterilisiert. Die in Ampullen abgefüllte Lösung wurde 3 Jahre bei Raumtemperatur gelagert und ist stabil.

## Beispiel 2

Analog Beispiel 1 wurde eine Diclofenac-Na Lösung hergestellt, jedoch wurden statt 200 mg Na-disulfit 200 mg Acetylcystein-HCl eingesetzt. Nach 3 Wochen Lagerung bei 81°C hat der Gehalt an Diclofenac-Na nur unwesentlich abgenommen. Eine Vorausberechnung nach der Methode von W. Grimm ließ eine Haltbarkeit von mehr als 3 Jahren erwarten.

## Beispiel 3

Analog Beispiel 1 wurde eine Diclofenac-Na Lösung hergestellt, jedoch wurden statt 200 mg Na-disulfit 200 mg Cystein-HCl eingesetzt. Nach 3 Wochen Lagerung bei 81°C hat der Gehalt an Diclofenac-Na nur unwesentlich abgenommen. Eine Vorausberechnung nach der Methode von W. Grimm ließ eine Haltbarkeit von mehr als 3 Jahren erwarten.

## EP 0 185 374 B1

### Beispiel 4

Es wurde eine Lösung nach Beispiel 2 hergestellt, jedoch wurden zusätzlich 3,3 g Prednisolon-21-phosphat-di-Natriumsalz zugesetzt. Die Lösung wurde in Ampullen zu 2 ml abgefüllt und bei 100°C 30 Minuten sterilisiert. Ein Streßversuch bei Lagerung bei 81°C läßt eine Haltbarkeit von mehr als 3 Jahren erwarten.

### Beispiel 5

Es wurde eine Lösung analog Beispiel 2 hergestellt. Zusätzlich wurden 524 mg Dexamethason-21-phosphat-di-Natriumsalz zugesetzt. Die Lösung wurde in Ampullen zu 2 ml abgefüllt und bei 100°C 30 Minuten sterilisiert. Ein Streßversuch bei Lagerung bei 81°C läßt eine Haltbarkeit von mehr als 3 Jahren erwarten.

### Patentansprüche für die Vertragsstaaten: BE CH DE FR GB IT LI LU NL SE

1. Flüssige Diclofenac-Zubereitungen, insbesondere für die parenterale Applikation, bestehend aus einer Lösung von Diclofenac oder eines seiner Salze und gegebenenfalls weiterer pharmazeutischen Wirkstoffen und Hilfsstoffen in einem Lösungsmittel, dadurch gekennzeichnet, daß das Lösungsmittel aus 10—70 Gew.-%, vorzugsweise 20—50 Gew.-%, einer Mischung aus a) Propylenglykol und b) Polyethylenglykol und 90—30 Gew.-%, vorzugsweise 80—50 Gew.-% Wasser besteht, wobei in dem Lösungsmittelgemisch das Gewichtsverhältnis von a) Propylenglykol zu b) Polyethylenglykol zwischen 9,5 : 0,5 und 0,5 : 9,5, vorzugsweise zwischen 3 : 1 und 1 : 3, liegt.

2. Diclofenac-Zubereitungen nach Anspruch 1, dadurch gekennzeichnet, daß in dem Lösungsmittelgemisch das Gewichtsverhältnis von a) Propylenglykol zu b) Polyethylenglykol zwischen 2 : 1 und 1 : 2 liegt.

3. Diclofenac-Zubereitungen nach einem der vorgehenden Ansprüche, dadurch gekennzeichnet, daß sie einen pH-Wert von 5,5—9, vorzugsweise 6—8,5 und insbesondere 6,5—8,0 aufweisen.

4. Diclofenac-Zubereitungen nach einem der vorgehenden Ansprüche, dadurch gekennzeichnet, daß sie als Polyethylenglykolkomponente ein Polyethylenglykol 400 enthalten.

5. Diclofenac-Zubereitungen nach einem der vorgehenden Ansprüche, dadurch gekennzeichnet, daß sie außerdem 0,1—5 Gew.-%, vorzugsweise 0,5—2 Gew.-%, eines Lokalanästhetikums von der Art des Lidocains enthalten.

6. Diclofenac-Zubereitungen nach Anspruch 5, dadurch gekennzeichnet, daß das Lokalanästhetikum Lidocain ist.

7. Diclofenac-Zubereitungen nach Anspruch 6, dadurch gekennzeichnet, daß sie einen pH-Wert von 7,5—8 aufweisen.

8. Diclofenac-Zubereitungen nach einem der vorgehenden Ansprüche, dadurch gekennzeichnet, daß sie zusätzlich 0,05—5 Gew.-% eines Reduktionsmittels als Stabilisator enthalten.

9. Diclofenac-Zubereitungen nach Anspruch 8, dadurch gekennzeichnet, daß sie als Reduktionsmittel Natriumdisulfit, Cysteinhydrochlorid, N-Acetylcysteinhydrochlorid oder Natriumthiosulfat enthalten.

10. Diclofenac-Zubereitungen nach einem der vorgehenden Ansprüche, dadurch gekennzeichnet, daß sie 1,5 bis 6 Gew.- Diclofenac enthalten.

11. Diclofenac-Zubereitungen nach einem der vorgehenden Ansprüche, dadurch gekennzeichnet, daß sie zusätzlich einen antiinflammatorisch wirksamen Arzneistoff, vorzugsweise ein Steroid, enthalten.

12. Diclofenac-Zubereitungen nach Anspruch 9, dadurch gekennzeichnet, daß sie im wesentlichen aus 3—4 Gew.-% Diclofenac-Natrium, etwa 24 Gew.-% Propylenglykol, etwa 18 Gew.-% Polyethylenglykol 400, etwa 1 Gew.-% Lidocainhydrochlorid, etwa 0,1 Gew.-% Natriumdisulfit, Rest Wasser bestehen, wobei die Lösungen mit Natronlauge auf einen pH-Wert von etwa 8 eingestellt sind.

### Patentansprüche für den Vetragsstaat: AT

1. Verwendung eines Lösungsmittel aus 10—70 Gew.-%, vorzugsweise 20—50 Gew.-%, einer Mischung aus a) Propylenglykol und b) Polyethylenglykol und 90—30 Gew.-%, vorzugsweise 80—50 Gew.-% Wasser wobei in dem Lösungsmittelgemisch das Gewichtsverhältnis von a) Propylenglykol zu b) Polyethylenglykol zwischen 9,5 : 0,5 und 0,5 : 9,5, vorzugsweise zwischen 3 : 1 und 1 : 3, liegt, zur Herstellung flüssiger Diclofenac-Zubereitungen, insbesondere für die parenterale Applikation.

2. Verwendung nach Anspruch 1, dadurch gekennzeichnet, daß in dem Lösungsmittelgemisch das Gewichtsverhältnis von a) Propylenglykol zu b) Polyethylenglykol zwischen 2 : 1 und 1 : 2 liegt.

3. Verwendung nach einem der vorgehenden Ansprüche, dadurch gekennzeichnet, daß das Lösungsmittel einen pH-Wert von 5,5—9, vorzugsweise 6—8,5 und insbesondere 6,5—8,0 aufweist

4. Verwendung nach einem der vorgehenden Ansprüche, dadurch gekennzeichnet, daß als Polyethylenglykolkomponente ein Polyethylenglykol 400 eingesetzt wird.

5. Verwendung nach einem der vorgehenden Ansprüche, dadurch gekennzeichnet, daß in dem Lösungsmittel außerdem 0,1—5 Gew.-%, vorzugsweise 0,5—2 Gew.-%, eines Lokalanästhetikums von der Art des Lidocains enthalten sind.

6. Verwendung nach Anspruch 5, dadurch gekennzeichnet, daß das Lokalanästhetikum Lidocain ist.

# EP 0 185 374 B1

7. Verwendung nach Anspruch 6, dadurch gekennzeichnet, daß das Lösungsmittel einen pH-Wert von 7,5—8 aufweist.

8. Verwendung nach einem der vorgehenden Ansprüche, dadurch gekennzeichnet, daß in dem Lösungsmittel zusätzlich 0,05—5 Gew.-% eines Reduktionsmittels als Stabilisator enthalten sind.

9. Verwendung nach Anspruch 8, dadurch gekennzeichnet, daß als Reduktionsmittel Natriumdisulfit, Cysteinhydrochlorid, N-Acetylcysteinhydrochlorid oder Natriumthiosulfat eingesetzt wird.

10. Verwendung nach einem der vorgehenden Ansprüche, dadurch gekennzeichnet, daß Diclofenac-Zubereitungen hergestellt werden, die 1,5 bis 6 Gew.-% Diclofenac enthalten.

11. Verwendung nach einem der vorgehenden Ansprüche, dadurch gekennzeichnet, daß Diclofenac-Zubereitungen hergestellt werden, die zusätzlich einen antiinflammatorisch wirksamen Arzneistoff, vorzugsweise ein Steroid, enthalten.

12. Verfahren zur Herstellung von flüssigen Diclofenac-Zubereitungen, insbesondere für die parenterale Applikation, dadurch gekennzeichnet, daß 3—4 Gew.-% Diclofenac-Natrium, etwa 24 Gew.-% Propylenglykol, etwa 18 Gew.-% Polyethylenglykol 400, etwa 1 Gew.-% Lidocainhydrochlorid und etwa 0,1 Gew.-% Natriumdisulfit in Wasser gelöst werden und die Lösungen mit Natronlauge auf einen pH-Wert von etwa 8 eingestellt werden.

## Revendications pour les Etats contractants: BE CH DE FR GB IT LI LU NL SE

1. Préparations liquides de diclofénac, en particulier pour administration parentérale, constituées d'une solution de diclofénac ou de l'un de ses sels, et éventuellement d'autres principes actifs et adjuvants pharmaceutiques dans un solvant, caractérisées en ce que le solvant est constitué de 10—70% en poids, de préférence de 20—50% en poids, d'un mélange a) de propylèneglycol et b) de polyéthylèneglycol, et de 90—30% en poids, de préférence de 80—50% en poids d'eau, et que, dans le mélange de solvants, le rapport pondéral propylène-glycol:polyéthyléneglycol est compris entre 9,5:0,5 et 0,5:9,5, de préférence entre 3:1 et 1:3.

2. Préparations de diclofénac selon la revendication 1, caractérisées en ce que, dans le mélange de solvants, le rapport pondéral entre a) le propylèneglycol et b) le polyéthylèneglycol est compris entre 2:1 et 1:2.

3. Préparations de diclofénac selon l'une des revendications précédentes, caractérisées en ce qu'elles ont un pH de 5,5—9, de préférence de 6—8,5 et en particulier de 6,5—8,0.

4. Préparations de diclofénac selon l'une des revendications précédentes, caractérisées en ce qu'elles contiennent en tant que composant polyéthylèneglycol un polyéthylèneglycol 400.

5. Préparations de diclofénac selon l'une des revendications précédentes, caractérisées en ce qu'elles contiennent en outre 0,1—5% en poids, de préférence 0,5—2% en poids, d'un anesthésique local du type de la lidocaïne.

6. Préparations de diclofénac selon la revendication 5, caractérisées en ce que l'anesthésique local est la lidocaïne.

7. Préparations de diclofénac selon la revendication 6, caractérisées en ce qu'elles ont un pH de 7,5—8.

8. Préparations de diclofénac selon l'une des revendications précédentes, caractérisées en ce qu'elles contiennent en outre, en tant que stabilisant, 0,05—5% en poids d'un réducteur.

9. Préparations du diclofénac selon la revendication 8, caractérisées en ce qu'elles contiennent en tant que réducteur du bisulfite de sodium, de chlorhydrate de cystéine, du chlorhydrate de N-acétylcystéine ou du thiosulfate de sodium.

10. Préparations de diclofénac selon l'une des revendications précédentes, caractérisées en ce qu'elles contiennent de 1,5 à 6% en poids de diclofénac.

11. Préparations de diclofénac selon l'une des revendications précédentes, caractérisées en ce qu'elles contiennent en outre un médicament à effet anti-inflammatoire, de préférence un stéroïdien.

12. Préparations de diclofénac selon la revendication 9, caractérisées en ce qu'elles sont constituées pour l'essentiel de 3—4% en poids de diclofénac sodique, d'environ 24% en poids de propylèneglycol, d'environ 18% en poids de polyéthylèneglycol 400, d'environ 1% en poids de chlorhydrate de lidocaïne, d'environ 0,1% en poids de bisulfite de sodium le reste étant constitué d'eau, les solutions étant ajustées à pH environ 8 avec de la lessive de soude.

## Revendications pour l'Etat contractant: AT

1. Utilisation d'un solvant constitué de 10—70% en poids, de préférence de 20—50% en poids, d'un mélange a) de propylèneglycol et b) de polyéthylèneglycol, et de 90—30% en poids, de préférence de 80—50% en poids d'eau, le rapport pondéral propylèneglycol:polyéthylèneglycol étant dans le mélange de solvants compris entre 9,5:0,5 et 0,5:9,5, de préférence entre 3:1 et 1:3, pour la réalisation de préparations liquides de diclofénac, en particulier pour administration parentérale.

2. Utilisation selon la revendication 1, caractérisée en ce que, dans le mélange de solvants, le rapport pondéral entre a) le propylèneglycol et b) le polyéthylèneglycol est compris entre 2:1 et 1:2.

3. Utilisation selon l'une des revendications précédentes, caractérisée en ce que le solvant a un pH de 5,5—9, de préférence de 6—8,5 et en particulier de 6,5—8,0.

4. Utilisation selon l'une des revendications précédentes, caractérisée en ce qu'on utilise en tant que composant polyéthylèneglycol un polyéthylène-glycol 400.

5. Utilisation selon l'une des revendications précédentes, caractérisée en ce que le solvant contient en outre 0,1—5% en poids, de préférence 0,5—2% en poids, d'un anesthésique local du type de la lidocaïne.

6. Utilisation selon la revendication 5, caractérisée en ce que l'anesthésique local est le lidocaïne.

7. Utilisation selon la revendication 6, caractérisé en ce que le solvant a un pH de 7,5—8.

8. Utilisation selon l'une des revendications précédentes, caractériséee en ce que le solvant contient en outre, en tant que stabilisant, 0,05—5% en poids d'un réducteur.

9. Utilisation selon la revendication 8, caractérisée en ce qu'on utilise comme réducteur du bisulfite de sodium, du chlorhydrate de cystéine, du chlorhydrate de N-acétylcystéine ou du thiosulfate de sodium.

10. Utilisation selon l'une des revendications précédentes, caractérisée en ce qu'on réalise des préparations de diclofénac contenant de 1,5 à 6% en poids de diclofénac.

11. Utilisation selon l'une des revendications précédentes, caractérisé en ce qu'on réalise des préparations du diclofénac qui contiennent en outre un médicament à effet anti-inflammatoire, de préférence un stéroïdien.

12. Procédé pour la réalisation de préparations liquides de diclofénac, en particulier pour administration parentérale, caractérisé en ce qu'on dissout dans l'eau 3—4% en poids de diclofénac sodique, environ 24% en poids de propylèneglycol, environ 18% en poids de polyéthylèneglycol 400, environ 1% en poids de chlorhydrate de lidocäine, et environ 0,1% en poids de bisulfite de sodium, les solutions étant ajustées à pH environ 8 à l'aide d'une lessive de soude.

## Claims for the Contracting States: BE CH DE FR GB IT LI LU NL SE

1. Liquid diclofenac preparations, especially for parenteral application, consisting of a solution of diclofenac or of one of its salts and optionally other pharmaceutical agents and adjuvants in a solvent, characterized in that the solvent consists of 10—70% by weight, preferably 20—50% by weight of a mixture of a) propylene glycol and b) polyethylene glycol and 90—30% by weight, preferably 80—50% by weight of water, the ratio by weight of a) propylene glycol to b) polyethylene glycol in the solvent mixture being between 9.5 : 0.5 and 0.5 : 9.5, preferably between 3:1 and 1:3.

2. Diclofenac preparations according to Claim 1, characterized in that in the solvent mixture the ratio by weight of a) propylene glycol to b) polyethylene glycol is between 2:1 and 1:2.

3. Diclofenac preparations according to one of the preceding claims, characterized in that they have a pH value of 5.5—9, preferably 6—8.5 and especially 6.5—8.0.

4. Diclofenac preparations according to one of the preceding claims, characterized in that they contain as polyethylene glycol component a polyethylene glycol 400.

5. Diclofenac preparations according to one of the preceding claims, characterized in that they also contain 0.1—5% by weight, preferably 0.5—2% by weight of a local anaesthetic of the lidocaine type.

6. Diclofenac preparations according to Claim 5, characterized in that the local anaesthetic is lidocaine.

7. Diclofenac preparations according to Claim 6, characterized in that they have a pH value of 7.5—8.

8. Diclofenac preparations according to one of the preceding claims, characterized in that they also contain 0.05—5% by weight of a reducing agent as stabilizer.

9. Diclofenac preparations according to Claim 8, characterized in that they contain as reducing agent sodium disulphite, cysteine hydrochloride, N-acetylcysteine hydrochloride or sodium thiosulphate.

10. Diclofenac preparations according to one of the preceding claims, characterized in that they contain 1.5 to 6% by weight diclofenac.

11. Diclofenac preparations according to one of the preceding claims, characterized in that they also contain a medicinal substance with antiinflammatory action, preferably a steroid.

12. Diclofenac preparations according to Claim 9, characterized in that they consist essentially of 3—4% by weight sodium diclofenac, about 24% by weight propylene glycol, about 18% by weight polyethylene glycol 400, about 1% by weight lidocaine hydrochloride, about 0.1% by weight sodium disulphite and the remainder water, the solutions being adjusted with caustic soda solution to a pH of about 8.

## Claims for the Contracting State: AT

1. Use of a solvent consisting of 10—70% by weight, preferably 20—50% by weight, of a mixture of a) propylene glycol and b) polyethylene glycol and 90—30% by weight, preferably 80—50% by weight, of water, the ratio by weight of a) propylene glycol to b) polyethylene glycol in the solvent mixture being between 9.5 : 0.5 and 0.5 : 9.5, preferably between 3:1 and 1:3, for the production of liquid diclofenac preparations, especially for parenteral application.

2. Use according to Claim 1, characterised in that in the solvent mixture the ratio by weight of a) propylene glycol to b) polyethylene glycol is between 2:1 and 1:2.

3. Use according to the preceding claims, characterised in that the solvent has a pH value of 5.5—9, preferably 6—8.5 and especially 6.5—8.0.

4. Use according to one of the preceding claims, characterised in that a polyethylene glycol 400 is used as polyethylene glycol component.

5. Use according to one of the preceding claims, characterised in that the solvent also contains 0.1—5% by weight, preferably 0.5—2% by weight, of a local anaesthetic of the lidocaine type.

6. Use according to Claim 5, characterised in that the local anaesthetic is lidocaine.

7. Use according to Claim 6, characterised in that the solvent has a pH value of 7.5—8.

8. Use according to one of the preceding claims, characterised in that the solvent also contains 0.05—5% by weight of a reducing agent as stabilizer.

9. Use according to Claim 8, characterised in that sodium disulphite, cysteine hydrochloride, N-acetylcysteine hydrochloride or sodium thiosulphate is used as reducing agent.

10. Use according to one of the preceding claims, characterised in that diclofenac preparations containing 1.5 to 6% by weight diclofenac are made.

11. Use according to one of the preceding claims, characterised in that diclofenac preparations are made which also contain a medicinal substance with antiinflammatory action, preferably a steroid.

12. Process for the production of liquid diclofenac preparations, especially for parenteral application, characterised in that 3—4% by weight sodium diclofenac, about 24% by weight propylene glycol, about 18% by weight polyethylene glycol 400, about 1% by weight lidocaine hydrochloride and about 0.1% by weight sodium disulphite are dissolved in water and the solutions are adjusted with caustic soda solution to a pH value of about 8.